# EUROPÄISCHE PATENTSCHRIFT

(11) **EP 3 073 244 B1**
(45) Veröffentlichungstag und Bekanntmachung des Hinweises auf die Patenterteilung: **03.10.2018**
(21) Anmeldenummer: 16401020.9
(22) Anmeldetag: 22.03.2016
(51) Int. Cl.: G01N 3/24, G01N 3/04, G01N 33/44

(54) **VORRICHTUNG UND EIN VERFAHREN ZUR BESTIMMUNG DER SCHERFESTIGKEIT UND DES SCHERMODULS VON VERBUNDWERKSTOFFEN**
DEVICE AND METHOD FOR DETERMINING THE SHEARING RESISTANCE AND THE SHEARING MODULUS OF COMPOSITE MATERIALS
DISPOSITIF ET PROCEDE DE DETERMINATION DE LA RESISTANCE AUX DECHIRURES ET DU MODULE DE RIGIDITE DE MATIERES COMPOSITES

(30) Priorität: 24.03.2015 DE 102015003689
(43) Veröffentlichungstag der Anmeldung: 28.09.2016
(73) Patentinhaber: Karlsruher Institut für Technologie, 76131 Karlsruhe (DE)
(72) Erfinder: Weidenmann, Kay André, 76137 Karlsruhe (DE); Haspel, Benedikt, 18055 Rostock (DE); Baumgärtner, Lisa, 74392 Freudental (DE)

(56) Entgegenhaltungen:
- WO-A1-2014/090298
- DE-U1- 8 336 167
- US-B1- 6 178 825
- K. SCHNEIDER ET AL: "Compression Shear Test (CST) - A Convenient Apparatus for the Estimation of Apparent Shear Strength of Composite Materials", APPLIED COMPOSITE MATERIALS, Bd. 8, Nr. 1, 1. Januar 2001 (2001-01-01), Seiten 43-62, XP055288490, NL ISSN: 0929-189X, DOI: 10.1023/A:1008919114960

## Beschreibung

Die Erfindung betrifft eine Vorrichtung und ein Verfahren zur Bestimmung der Scherfestigkeit und des Schermoduls von Verbundwerkstoffen.

Es ist bekannt, zur Bestimmung der interlaminaren Scherfestigkeit von faserverstärkten Kunststoffen einen sogenannten Kurzbiegeversuch nach **[1]** zu verwenden. Darin wird verwiesen, dass die scheinbare interlaminare Scherfestigkeit mit Hilfe des Kurzbiegeversuchs nur dann ermittelt werden kann, wenn ein Versagen durch interlaminare Scherung hervorgerufen wird. Dies ist der Fall, wenn ein Riss in der Mittelebene und seitlich eine gegenseitige Verschiebung der oberen und unteren Hälfte der Probe zu erkennen sind. Des Weiteren wird in **[1]** eine Probengröße vorgeschrieben. Es sind zwar prinzipiell verschiedene Probengrößen prüfbar, aber immer mit einem vorgegebenen Verhältnis der Probengeometrie, wobei die Auflagerabstand ebenfalls in festem Verhältnis zur Probengeometrie stehen müssen. Voraussetzung hierfür ist aber, dass die Probe länger als die Stützweite der Auflager ist. Zugleich darf die Probe nicht zu lang sein, da ab einem vom Werkstoff abhängigen Verhältnis von Länge zu Breite kein Scher- sondern ein Biegeversagen auftritt.

Oftmals tritt jedoch bei der in **[1]** vorgegebenen Durchführung zur Bestimmung der scheinbaren interlaminaren Scherfestigkeit (Interlaminar Shear Strenth ILSS) kein interlaminares Schubversagen auf, weil die Druckruckfinne und die Auflager hohe Druckspannungen in der Probe erzeugen. Hieraus resultieren unzulässige Spannungskonzentrationen sowie Biegemomente, wodurch die Materialfestigkeit überschritten wird. Das Überschreiten der Materialfestigkeit tritt ein, noch bevor die beabsichtigte interlaminare Scherung eingesetzt hat. Hinzu kommt das die ermittelten Werte für die interlaminare Scherfestigkeit von der Probengeometrie abhängig sind. Ein Vergleich der interlaminaren Scherfestigkeit verschiedener Werkstoffe vorzugsweise Laminate setzt somit eine Gleiche oder wenigstens ähnliche Probengeometrie voraus.

Daher kann der Kurzbiegeversuch lediglich zur Qualitätssicherung eingesetzt werden. Eine Wahl der Scherebenen ist hier nicht möglich.

In diesem Zusammenhang wird als Laminat ein Werkstoff definiert, welcher aus zwei oder mehreren flächig miteinander verbundenen Schichten besteht. Diese Schichten können aus gleichen oder unterschiedlichen Materialien bestehen. Die einzelnen Schichten sind hierzu vorzugsweise flächig miteinander verklebt oder verweist. Ein Laminat aus unterschiedlichen Werkstoffen wird auch als Hybrid bezeichnet. Laminate aus faserverstärkten Kunststoffen sind feste, leichte und frei gestaltbare Werkstoffe aus in Kunstharz eingebetteten Fasern. Sie werden in der Luftfahrt, im Boots- und Automobilbau und in vielen weiteren Bereichen eingesetzt, in denen hochfeste und leichte Materialien benötigt werden.

Am Leibniz-Institut für Polymerforschung Dresden e. V. wurde ein sog. Compression Shear Test (CST)entwickelt. Eine Vorrichtung zur Durchführung des CST wird in **[2]** offenbart. Hier wird in einer Prüfvorrichtung eine Probe, zwischen zwei seitlichen ortsfesten Halterungen geklemmt, wobei ein Teil der Probe auf einer ersten Schneide aufliegt. Der restliche Teil der Probe wird von einer weiteren zweiten Schneide, die an der Vorrichtung schwenkbar gelagert ist, belastet. Die Lagerung samt Schneide ist in ihrer Höhe verstellbar, sodass die Schneide bei beliebigen Probenabmessungen zu Beginn des Versuchs horizontal ist. Nach dem Einspannen der Probe wird mit Hilfe einer Druckfinne auf die obere Schneide gedrückt. Die interlaminare Scherfestigkeit lässt sich demnach über die maximal aufgebrachte Kraft des Druckkopfes und den Hebelarm der Schneide bestimmen. In der Vorrichtung können beliebige Probengrößen geprüft werden, jedoch ist das Verhältnis von Probenhöhe zu Probenbreite nicht beliebig, da bei zu großen Verhältnissen kein Scherversagen, sondern ein Druckversagen erfolgt.

**[3]** offenbart eine Vorrichtung zur Durchführung eines Schneidscherversuchs zur Ermittlung der interlaminaren Scherfestigkeit von faserverstärkten Kunststoffen. Der prinzipielle Aufbau und Durchführung ähnelt dem Compression Shear Test (CST) welcher am Leibniz-Institut für Polymerforschung Dresden e.V. entwickelt wurde. Jedoch wirkt in der Vorrichtung nach **[3]** statt einem Drehmoment eine Druckkraft auf die Probe. Hierfür kann die obere Metallschneide in vertikaler Richtung verschoben werden. In der Schneidschervorrichtung können beliebige Probengrößen geprüft werden. Prinzipiell sinkt die Standardabweichung der gemessenen interlaminaren Scherfestigkeit durch ein Vergrößern der Probenhöhe h. Offenbart wird auch eine optimale Probehöhe h von 10 mm, bei einer Probenbreite von 20 mm und eine Probendicke d von mindestens 1,5 mm. Bei Werkstoffen mit geringen Druckfestigkeiten bei gleichzeitig hohen interlaminaren Scherfestigkeiten ist jedoch die Probehöhe h zu reduzieren beziehungsweise die Probendicke d zu erhöhen, sodass die Probe noch durch interlaminare Scherung versagt. Dementsprechend ist auch hier das Verhältnis von Probenhöhe h zu Probendicke d nicht beliebig, da bei zu großen Verhältnissen kein Scherversagen, sondern ein Druckversagen erfolgt.

In **[4]** wird eine Vorrichtung für die Scherfestigkeitsprüfung von Proben offenbart, die in Fügeverfahren hergestellt sind. Dies sind insbesondere geschweißte, geklebte, gelötete, geschraubte oder genietete Proben. Die Proben sind in einer bestimmten Scherebene mit einer Scherkraft belastbar. Die Auswahl der Scherebene erfolgt über das Einbringen von Abstandsblechen. Die Vorrichtung ermöglicht die Messung der Scherfestigkeit der Proben entlang dieser Scherebene. Die Vorrichtung sieht jedoch nicht die direkte Messung des Schwermoduls vor.

**[5]** offenbart ebenfalls eine Schubprüfvorrichtung zur mechanischen Werkstoffprüfung. Die Schubprüfvorrichtung weist zwei Klemmblöcke zum Einspannen je eines Endes eines mit einer Prüfkraft belasteten Prüfkörpers auf. Beim Aufbringen einer Prüfkraft dehnt sich der Prüfkörper quer zur Prüfkraftrichtung frei, ohne dass querdehnungsinduzierte Normalspannungen im Prüfquerschnitt entstehen. Die offenbarte Schubprüfvorrichtung dient der Bestimmung der intralaminaren Schereigenschaften eines Verbundwerkstoffes, d.h. der Eigenschaften, die der zu untersuchende Verbundwerkstoff, z.B. ein faserverstärkter Kunststoff, innerhalb der Laminierungsschichten aufweist. Die Vorrichtung dient nicht der Bestimmung der interlaminaren Schereigenschaften des Verbundwerkstoffes. Die interlaminaren Schereigenschaften beschreiben die Festigkeit zwischen einzelnen Laminierungsschichten aus denen Ein Verbundwerkstoff aufgebaut ist. Diese Eigenschaften steuern beispielsweise das Delaminierungsverhalten eines Verbundwerkstoffes.

In **[6]** wird eine Vorrichtung zur Bestimmung der Schereigenschaften speziell von Sandwichmaterialien mit Wabenkernen offenbart. Diese Materialien setzen sich aus einer oberen und unteren Ebene zusammen, die durch einen Kern aus wabenförmigen Röhren miteinander verbunden sind. Die offenbarte Vorrichtung besteht aus zwei Grundkörpern in einem festen Abstand zueinander. Er ist durch zwei Abstandshalter vorgegeben und entspricht der Dicke des zu untersuchenden Sandwichmaterials. Zwischen den Grundkörpern der Vorrichtung wird eine Probe aus dem zu untersuchenden Sandwichmaterial eingespannt. Die Lasteinwirkung erfolgt über einen der Grundkörper, senkrecht zum Wabenkern der Probe. Der andere Grundkörper verbleibt fest in seiner Ausgangsposition. Die Abstandshalter sind drehbar gelagert und werden bei Lasteinwirkung auf den beweglichen Grundkörper mitbewegt. Die drehbar gelagerten Abstandhalter sorgen dafür, dass die Probe über die gesamte Breite des Wabenkerns geschert wird, ohne dass die Röhren innerhalb des Wabenkerns an einer bestimmten Stelle abknicken.

Ausgehend davon liegt eine Aufgabe der Erfindung darin, eine Vorrichtung und ein Verfahren zur mechanischen Werkstoffprüfung vorzuschlagen, welche die aufgeführten Nachteile und Einschränkungen vermeidet.

Im speziellen soll die Vorrichtung eine Versuchsdurchführung für beliebige Probengrößen bzw. beliebige Verhältnisse h/d von Probenhöhe h zu Probendicke d ermöglichen, wobei die Scherebene über die gesamte Probendicke d frei wählbar ist.

Da die Prüfkörper eines laminaren hybriden Werkstoffes häufig nur in einer vorbestimmten Geometrie (Dicke) hergestellt werden, ist es vorteilhaft, wenn eine variierbare Probengröße in der Vorrichtung geprüft werden kann. Eine variierbare Probengröße hat zudem den Vorteil, dass bei Werkstoffen, die während der Prüfung nicht durch interlaminare Scherung, sondern durch einen anderen Versagensmodus versagen, in ihrer Probenhöhe reduziert werden können bis interlaminares Scherversagen vor einem Druckversagen auftritt. Insbesondere bei zähen Werkstoffen ist es von Vorteil, wenn ein langer Verfahrweg der Schervorrichtung möglich ist, sodass eine Probe über ihre gesamte Höhe abgeschert werden kann.

Insbesondere soll die Effizienz der Prüfvorrichtung betreffend der Einrichtungsdauer pro Versuch durch eine einfachere Handhabung gesteigert werden. Ferner wird ein spannungsfreier Proben- bzw. Prüfkörpereinbau zu Versuchsbeginn ermöglicht, wodurch die Aussagefähigkeit der Ergebnisse verbessert wird. Außerdem kann und wird der Spannungszustand während der Versuchsdurchführung bewertet.

Gelöst wird diese Aufgabe im Hinblick auf die Prüfvorrichtung mit einer Vorrichtung mit den Merkmalen nach Anspruch 1 und im Hinblick auf das Verfahren durch die Verfahrensschritte gemäß dem Anspruch 7. Die Unteransprüche geben vorteilhafte Ausgestaltungen der Erfindung an.

Zur Lösung der Aufgabe wird eine Vorrichtung vorgeschlagen, welche einen verfahrbaren oberen Grundkörper und einen ortsfesten unteren Grundkörper umfasst.

Ferner ist der obere Grundkörper durch parallel zur Scherebene ausgerichtete Führungsstangen mit dem unteren Grundkörper gekoppelt, wobei der erste, obere Grundkörper, parallel zur Scherebene entlang der Führungsstangen verfahrbar ist. Hierzu ist der obere Grundkörper mit einer Lagerung, vorzugsweise einer Linearlagerung, gegenüber den Führungsstangen ausgestattet.

Im Probenraum bzw. dem Freiraum zwischen dem oberen Grundkörper und unteren Grundkörper wird die Probe bzw. der Probenkörper positioniert.

Die Vorrichtung so ausgestaltet, dass der Probenraum ohne Demontage der Vorrichtung oder Teile der Vorrichtung zugänglich ist. Somit wird ein schneller und unkomplizierter Probenwechsel gewährleistet.

Am oberen Grundkörper seitlich zur Probe und parallel zur Scherebene ist eine erste Abstützplatte angebracht. Ebenso ist eine weitere Abstützplatte an dem unteren Grundkörper seitlich zur Probe und parallel zur Scherebene vorgesehen. Insbesondere liegen die Abstützplatten sich spiegelbildlich gegenüber und sind durch die Dicke d der Probe beabstandet voneinander. Die Scherebene ist orthogonal zum Verfahrweg über die gesamte Dicke d der Probe beliebig einstellbar, wobei die Scherebene immer parallel zum Verfahrweg ist. Mittels zusätzlicher Führungsschienen sind die Abstützplatten orthogonal zur Scherebene jeweils unabhängig von dem ersten oberen Grundkörper oder dem zweiten unteren Grundkörper verschiebbar. Die seitlich zur Probe angebrachten Abstützplatten sind mittels Führungsschienen mit den Grundkörpern über jeweils eine zugehörige Gewindestange verbunden. Durch das Ein- bzw. Ausdrehen der Gewindestangen werden die seitlichen Führungsschienen zusammen mit den Abstützplatten orthogonal zur Scherebene verfahren und somit die Scherebene an der Probe eingestellt. Ferner ist an dem oberen Grundkörper eine Druckplatte und an dem unteren Grundkörper eine weitere Druckplatte vorgesehen, wobei die Druckplatten orthogonal zur Scherebene ausgerichtet sind und zur Einleitung der Prüfkraft in die Probe ausgestaltet sind. Die Druckplatte am oberen Ende der Probe sowie die Druckplatte am unteren Ende der Probe liegen hierzu flächig auf der Probe auf. Ferner ist vorgesehen, dass der Verfahrweg mit Hilfe eines Sensors, vorzugsweise eines Wegaufnehmers direkt an der Probe ermittelt wird.

Nach Anspruch 7 umfasst das Verfahren zur Bestimmung der interlaminaren Scherfestigkeit und des Schermoduls von faserverstärkten Kunststofflaminaten folgende Verfahrensschritte:
Die Probe wird in dem Probenraum zwischen dem ersten verfahrbaren oberen Grundkörper mit einer zugehörigen Druckplatte und einem zweiten ortsfesten unteren Grundkörper mit einer zugehörigen Druckplatte positioniert. Die Scherebene wird anschliessend über das Ein- bzw. Ausdrehen der Gewindestangen eingestellt, wobei die seitlichen Führungsschienen zusammen mit den Abstützplatten gemeinsam verfahren werden. Die Prüfkraft F wird über den oberen Grundkörper mit der zugehörigen Druckplatte in die Probe eingebracht und simultan die Prüfkraft F und der Verfahrweg s an der Probe gemessen. Aufgezeichnet wird vorzugsweise die Prüfkraft F über den Verfahrweg s als bis zum Versagen des Körpers ansteigende Prüfkraft-Verfahrweg-Kurve, aus der in herkömmlicher Weise unter Einbeziehung der Probendaten und -abmessungen die Scherspannungs-Scherungs-Kurve ableitbar ist. Das Verfahren umfasst daher vorzugsweise auch eine Bestimmung der interlaminaren Scherfestigkeit aus der maximalen Prüfkraft (F) bezogen auf die gescherte Probenfläche bevorzugt an der oder um die Scherebene. Ferner umfasst das Verfahren vorzugsweise eine Bestimmung des Schermoduls aus der Steigung der Scherspannungs-Scherungs-Kurve welche aus der Prüfkraft-Verfahrweg-Kurve kontinuierlich abgeleitet wird.

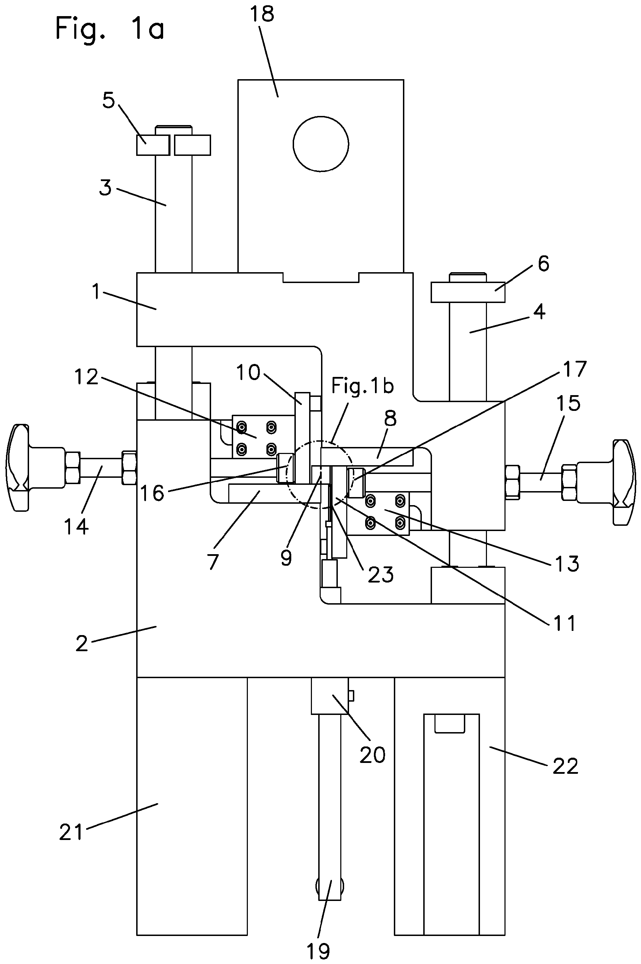

Nachfolgend wird die Erfindung anhand von Ausführungsformen mit Figuren näher erläutert. Es zeigen
**Fig.1a** eine schematische Darstellung der erfindungsgemäßen Vorrichtung sowie

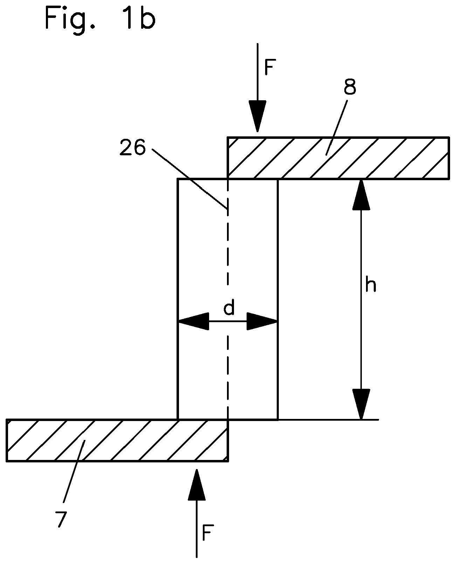

**Fig.1b** eine schematische Darstellung des Probenraums mit einer Probe

Anhand von **Fig.1a** soll zunächst der grundlegende Aufbau und die Funktionsweise der der Vorrichtung gezeigt werden.

Die Vorrichtung umfasst einen oberen Grundkörper **1** und einen unteren Grundkörper **2.** Die Führungsstangen **3, 4** sind im unteren Grundkörper **2** formschlüssig verbunden, vorzugsweise mit einer Presspassung. Im oberen Grundkörper **1** ist jeweils eine Lagerung, vorzugsweise ein Linearlager pro Führungsstange **3, 4** integriert, welche ein reibungsarmes Gleiten des oberen Grundkörpers **1** auf den Führungsstangen **3, 4** ermöglichen. Durch Klemmringe **5, 6** wird der Verfahrweg des oberen Grundkörpers nach oben begrenzt.

Zudem ist an den beiden Grundkörpern **1, 2** jeweils eine zugehörige Druckplatte **7, 8** befestigt. Die Druckplatten leiten die durch eine nicht dargestellte Prüfmaschine erzeugte Kraft F (Prüfkraft, Belastung) mittels Adapter **18** in einem offenen Laststrang auf die Probe **9** weiter, indem der obere Grundkörper **1** bzw. die Druckplatte **8** an die Probe **9** gefahren wird.
Mit Hilfe von zwei Probenführungseinstellmitteln, vorzugsweise zwei Gewindestangen **14, 15** und einem entsprechendem Gewinde im oberen und unteren Grundkörper **1, 2** werden die seitlichen Führungsschienen **12, 13** mit den Abstützplatten **10, 11** gegen die Probe **9** positioniert und verhindern somit ein Verkippen der Probe **9** während der Versuchsdurchführung. Aus der Positionierung der Abstützplatten heraus, wirkt jedoch keine korrektive Kraft auf die Probe **9.**

Durch die Führungsschienen **12, 13** wird zudem gewährleistet, dass die seitlichen Abstützplatten **10, 11** stets senkrecht ausgerichtet sind und einer potentiellen Bewegung der Probe **9** in Richtung des Kraftflusses folgen.

Die Führungsschienen **12, 13** sind wiederum am oberen Grundkörper **1** beziehungsweise unteren Grundkörper **2** befestigt. Durch die aufgeschraubten Gewindeköpfe **16, 17** wird die Kontaktfläche zwischen der Gewindestange **14, 15** und den seitlichen Abstützplatte **10, 11** vergrößert.

Während der Scherprüfung wird der Verfahrweg s direkt an der Probe **9** mit Hilfe eines Wegaufnehmers **19** ermittelt und simultan die Prüfkraft F aufgezeichnet.

Durch die aufgewandte Prüfkraft F zusammen mit der vorher gemessenen Scherfläche der Probe **9** wird die Scherfestigkeit ermittelt. An dem Wegaufnehmer **19** ist eine Klemme **20** befestigt, in der ein Magnet integriert ist. Mit Hilfe der Klemme **20** wird der Wegaufnehmer **19** von unten an dem unteren Grundkörper **2** positioniert und allein durch die Magnetkraft fixiert. Aufgrund der großen Länge des Wegaufnehmers **19** müssen bei Verwendung dessen zusätzlich zwei Sockel **21, 22** am unteren Grundkörper **2** angebracht werden. Zudem wird ein spezieller Aufsatz **23** als Wegtaster verwendet, wodurch der Wegaufnehmer **19** insbesondere bei sehr dünnen Proben **9** zum Einsatz kommt. Hierdurch wird es möglich die Scherung der Probe **9** bereits während der Versuchsdurchführung zu ermitteln und zu bewerten. Abschließend wird der Schermodul aus Scherspannung und Scherung der Probe **9** bestimmen.

**Fig.1b** zeigt eine schematische Darstellung des Probenraums mit einer Probe **9,** wobei die Probe **9** kraftschlüssig mit einer oberen Druckplatte **8** und einer unteren Druckplatte **7** eines offenen Laststranges einer nicht dargestellten Prüfmaschine verbunden ist. Zum Erzeugen einer Prüfkraft F wird der obere Kraftangriffspunkt der Druckplatte **8** mit der Probe **9** relativ zum unteren Kraftangriffspunkt der Druckplatte **7** mit der Probe **9** geradlinig entlang der Scherebene **26** bewegt.

Die obige Beschreibung soll der Veranschaulichung dienen und nicht einschränkend sein. Beispielsweise können die oben beschriebenen Beispiele (oder einer oder mehrere Aspekte davon) miteinander in Kombination verwendet werden. Andere Ausführungsformen können beispielsweise vom Durchschnittsfachmann nach Durchsicht der obigen Beschreibung verwendet werden.

Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.

In diesem Dokument werden die Ausdrücke "beinhalten(d)"/"enthalten(d)" und "in welchem" als Leichtsprachäquivalente der entsprechenden Ausdrücke "umfassen(d)" und "wobei" verwendet. Außerdem sind in den folgenden Ansprüchen die Begriffe "beinhalten(d)"/"enthalten(d)" und "umfassen(d)" nicht abschliessend, das heißt, ein System, eine Vorrichtung, ein Artikel oder ein Verfahren, das Bestandteile umfasst, die über die nach einem solchen Begriff in einem Anspruch aufgeführten Bestandteile hinausgehen, wird als noch in den Schutzbereich des Anspruchs fallend erachtet.

Ferner werden in den folgenden Ansprüchen die Begriffe "erste(r)", "zweite(r)" und "dritte(r)" etc. lediglich als Kennzeichen verwendet und beabsichtigen nicht zahlenmäßige Forderungen an ihre Objekte zu stellen.

### Literatur

[1] DIN ISO 14130 Faserverstärkte Kunststoffe - Bestimmung der scheinbaren interlaminaren Scherfestigkeit nach dem Dreipunktverfahren mit kurzem Balken
[2] K.Schneider, B.Lauke, W.Beckert: Compression Shear Test (CST) - A Convenient Apparatus for the Estimation of Apparent Shear Strength of Composite Materials, Applied Composite Materials 8: 43-62, 2001
[3] Drechsler, K.: Beitrag zur Gestaltung und Berechnung von Faserverbundwerkstoffen mit dreidimensionaler Textilverstärkung. Universität Stuttgart, Dissertation, 1992
[4] DE 83 36 167 U1 29. April 1993 (1993-04-29)
[5] WO 2014/090298 A1 (IMA Materialforschung und Anwendungstechnik GmbH [DE]) 19. Juni 2014 (2014-06-19)
[6] US 6 178 825 B1 (Chang Dick J [US] et al.) 30. Januar 2001 (2001-01-30)

### Bezugszeichenliste

- 1: oberer Grundkörper
- 2: unterer Grundkörper
- 3: Führungsstange
- 4: Führungsstange
- 5: Klemmring
- 6: Klemmring
- 7: Druckplatte
- 8: Druckplatte
- 9: Probe
- 10: seitliche Abstützplatten
- 11: seitliche Abstützplatten
- 12: Führungsschienen
- 13: Führungsschienen
- 14: Gewindestangen
- 15: Gewindestangen
- 16: Gewindekopf
- 17: Gewindekopf
- 18: Adapter
- 19: Wegaufnehmer
- 20: Klemme
- 21: Sockel
- 22: Sockel
- 23: Wegtaster
- 24: Traverse
- 25: Kraftmessdose
- 26: Scherebene
- 27: Verfahrweg
- 28: Probendicke / Dicke der Probe (d)
- 29: Probenhöhe / Höhe der Probe (h)

## Patentansprüche

1. Vorrichtung zur Bestimmung der interlaminaren Scherfestigkeit und des Schermoduls einer Probe (**9**) aus faserverstärkten Kunststofflaminaten an einer Scherebene (**26**), umfassend
a) einen verfahrbaren oberen Grundkörper (**1**) und einen ortsfesten unteren Grundkörper (**2**),
b) wobei die Probe zwischen dem oberen Grundkörper (**1**) und dem unteren Grundkörper (**2**) in einem Probenraum positioniert ist,
c) wobei an dem oberen Grundkörpern (**1**) seitlich zur Probe (**9**) und parallel zur Scherebene (**26**) eine Abstützplatte (1**0)** und an dem unteren Grundkörper (**2**) seitlich zur Probe (**9**) und parallel zur Scherebene (**26**) und gegenüberliegend der Abstützplatte (**10**) eine weitere Abstützplatte **(11)** durch die Dicke (d) der Probe (**9**) beabstandet vorgesehen ist,
d) wobei die Scherebene (**26**) orthogonal zum Verfahrweg (**27**) über die gesamte Dicke (d) der Probe (**9**) beliebig einstellbar ist wobei die Scherebene (**26**) parallel zum Verfahrweg (**27**) liegt,
e) wobei an dem oberen Grundkörper (**1**) eine Druckplatte (**7**) und an dem unteren Grundkörper (**2**) eine Druckplatte (**8**) befestigt ist, wobei die Druckplatten (**7, 8**) orthogonal zur Scherebene (**26**) ausgerichtet sind und zur Einleitung einer Kraft in die Probe (**9**) ausgebildet sind,
**dadurch gekennzeichnet, dass**
f) die Abstützplatten (**10, 11**) mittels Führungsschienen **(12, 13)** orthogonal zur Scherebene (**26**) jeweils unabhängig vom oberen Grundkörper (**1**) oder dem unteren Grundkörper (**2**) verschiebbar sind.

2. Vorrichtung nach Anspruch 1, **dadurch gekennzeichnet, dass** die seitlich zur Probe (**9**) angebrachten Abstützplatten (**11**, **12**) mittels der Führungsschienen (**12, 13**) mit den Grundkörpern (**1, 2**) über jeweils eine Gewindestange (**14**, **15**) verbunden sind.

3. Vorrichtung nach Anspruch 2, **dadurch gekennzeichnet, dass** durch das Ein- bzw. Ausdrehen der Gewindestangen (**14, 15**) die seitlichen Führungsschienen (**12, 13**) zusammen mit den Abstützplatten (**10, 11**) orthogonal zur Scherebene (**26**) verfahrbar sind.

4. Vorrichtung nach Anspruch 3, **dadurch gekennzeichnet, dass** der obere Grundkörper (**1**) durch parallel zur Scherebene (**26**) ausgerichtete Führungsstangen (**3, 4**) mit dem unteren Grundkörper (**2**) gekoppelt ist, wobei der Grundkörper (**1**) parallel zur Scherebene (**26**) entlang der Führungsstangen (**3, 4**) verfahrbar ist.

5. Vorrichtung nach Anspruch 4, **dadurch gekennzeichnet, dass** die Druckplatte (**7**) am oberen Ende und die Druckplatte (**8**) am unteren Ende der Probe (**9**) flächig aufliegen.

6. Vorrichtung nach Anspruch 5, **dadurch gekennzeichnet, dass** der Verfahrweg (**27**) mit Hilfe eines Wegaufnehmers (**19**) direkt an der Probe (**9**) ermittelt wird.

7. Verfahren zur Verwendung der Vorrichtung nach Anspruch 1 zur Bestimmung der interlaminaren Scherfestigkeit und des Schermoduls von faserverstärkten Kunststofflaminaten, umfassend folgende Verfahrensschritte:
a) Positionieren einer Probe (**9**) zwischen einem ersten verfahrbaren oberen Grundkörper (**1**) mit einer zugehörigen Druckplatte (**8**) und einem zweiten ortsfesten unteren Grundkörper (**2**) mit einer zugehörigen Druckplatte (**7**),
b) Einstellen der Scherebene (**26**) durch das Ein- bzw. Ausdrehen der Gewindestangen (**14, 15**) wobei die seitlichen Führungsschienen **(12, 13)** zusammen mit den Abstützplatten (**10, 11**) orthogonal zur Scherebene (**26**) verfahren,
c) Aufbringen der Prüfkraft F und simultanes messen des Verfahrwegs (s) und der Prüfkraft (F),
d) Bestimmung der interlaminaren Scherfestigkeit aus der maximalen Prüfkraft (F) bezogen auf die gescherte Probenfläche,
e) Bestimmung des Schermoduls aus der Steigung der Scherspannungs-Scherungs-Kurve welche aus der Prüfkraft-Verfahrweg-Kurve kontinuierlich abgeleitet wird.

## Claims

1. Device for determining the interlaminar shearing resistance and the shearing modulus of a sample (9) of fibre-reinforced plastic laminates at a shearing plane (26), comprising
a) a movable upper base body (1) and a fixed-position lower base body (2),
b) wherein the sample is positioned between the upper base body (1) and the lower base body (2) in a sample space,
c) wherein, at the upper base body (1), laterally to the sample (9) and parallel to the shear plane (26), a support plate (10) is provided, and at the lower base body (2), laterally to the sample (9), parallel to the shear plane (26), and opposite the support plate (10), a further support plate (11) is provided, spaced apart by the thickness (d) of the sample (9),
d) wherein the shearing plane (26) can be adjusted as desired orthogonally to the movement path (27) over the entire thickness (d) of the sample (9), wherein the shearing plane (26) lies parallel to the travel path (27),
e) wherein a pressure plate (7) is secured to the upper base body (1), and a pressure plate (8) is secured to the lower base body (2), wherein the pressure plates (7, 8) are aligned orthogonally to the shearing plane (26) and are configured such as to introduce a force into the sample (9),
**characterised in that**
f) the support plates (10, 11) can be relocated by means of guide rails (12, 13) orthogonally to the shearing plane (26) in each case independently of the upper base body (1) or of the lower base body (2).

2. Device according to claim 1, **characterised in that** the support plates (11, 12) located laterally to the sample (9) by means of the guide rails (12, 13) are connected to the base bodies (1, 2), in each case by means of a threaded rod (14, 15).

3. Device according to claim 2, **characterised in that**, by the inward and outward rotation of the threaded rods (14, 15), the lateral guide rails (12, 13) can be moved together with the support plates (10, 11) orthogonally to the shearing plane (26).

4. Device according to claim 3 **characterised in that** the upper base body (1) is coupled by means of guide rods (3, 4), aligned parallel to the shearing plane (26), to the lower base body (2), wherein the base body (1) can be moved parallel to the shearing plane (26) along the guide rods (3, 4)

5. Device according to claim 4, **characterised in that** the pressure plate (7) lies in flat surface configuration at the upper end of the sample (9), and the pressure plate (8) at its lower end.

6. Device according to claim 5, **characterised in that** the travel path (27) is determined directly on the sample (9) with the aid of a path sensor (19).

7. Method for using the device according to claim 1 for the determination of the interlaminar shearing resistance and the shearing modulus of fibre-reinforced plastic laminates, comprising the following method steps:
a) positioning a sample (9) between a first movable upper base body (1) with an associated pressure plate (8), and a second fixed-position lower base body (2) with an associated pressure plate (7),
b) adjusting the shearing plane (26) by the inward and outward rotation respectively of the threaded rods (14, 15), wherein the lateral guide rails (12, 13) move together with the support plates (10, 11) orthogonally to the shearing plane (26),
c) applying the testing force F and simultaneously measuring the travel path (s) and the testing force (F),
d) determination of the interlaminar shearing resistance from the maximum testing force (F) related to the sheared sample surface,
e) determination of the shearing modulus from the rise of the shear-tension-shearing curve which is continually derived from the testing force-travel path curve.

## Revendications

1. Dispositif permettant de déterminer la résistance au cisaillement inter-laminaire et le module de cisaillement d'un échantillon (9) réalisé en des stratifiés en matériau synthétique renforcé par des fibres dans un plan de cisaillement (26), comprenant :
a) un corps de base supérieur mobile (1) et un corps de base inférieur fixe (2),
b) l'échantillon étant positionné entre le corps de base supérieur (1) et le corps de base inférieur (2) dans une chambre à échantillon,
c) une plaque d'appui (10) étant montée sur le corps de base supérieur (1) latéralement à l'échantillon (9) et parallèlement au plan de cisaillement (26), et une autre plaque d'appui (11) séparée par l'épaisseur (d) de l'échantillon (9), opposée à la plaque d'appui (10) étant montée sur le corps de base inférieur (2) latéralement par rapport à l'échantillon (9) et parallèlement au plan de cisaillement (26),
d) le plan de cisaillement (26) étant réglable à volonté perpendiculairement à la course de déplacement (27) sur la totalité de l'épaisseur (d) de l'échantillon (9), le plan de cisaillement (26) étant parallèle à la course de déplacement (27),
e) sur le corps de base supérieur (1) étant fixée une plaque de compression (7) et sur le corps de base inférieur (2) étant fixée une plaque de compression (8), les plaques de compression (7, 8) étant orientées perpendiculairement au plan de cisaillement (26), et réalisées pour permettre d'appliquer une force dans l'échantillon (9), **caractérisé en ce que**
f) les plaques d'appui (10, 11) sont mobiles en translation au moyen de rails de guidage (12, 13) perpendiculairement au plan de cisaillement (26) respectivement indépendamment du corps de base supérieur (1) ou du corps de base inférieur (2).

2. Dispositif conforme à la revendication n1,
**caractérisé en ce que**
les plaques d'appui (11, 12) montées latéralement par rapport à l'échantillon (9) sont reliées aux corps de base (1, 2) au moyen des rails de guidage (12, 13) par l'intermédiaire d'une tige filetée (14, 15) respective.

3. Dispositif conforme à la revendication 2,
**caractérisé en ce que**
par la rotation dans un sens ou dans l'autre, des tiges filetées (14, 15), les rails de guidage latéraux (12, 13) peuvent être déplacés avec les plaques d'appui (10, 11) perpendiculairement au plan de cisaillement (26).

4. Dispositif conforme à la revendication 3,
**caractérisé en ce que**
le corps de base supérieur (1) est couplé au corps de base inférieur (2), par l'intermédiaire de tiges de guidage (3, 4) orientées parallèlement au plan de cisaillement (26), le corps de base (1) pouvant être déplacé parallèlement au plan de cisaillement (26) le long des tiges de guidage (3, 4).

5. Dispositif conforme à la revendication 4,
**caractérisé en ce que**
la plaque de compression (7) est appliquée à plat à l'extrémité supérieure de l'échantillon (9) et la plaque de compression (8) est appliquée à plat à l'extrémité inférieure de l'échantillon (9).

6. Dispositif conforme à la revendication 5,
**caractérisé en ce que**
la course de déplacement (27) est déterminée directement sur l'échantillon (9) à l'aide d'un capteur de déplacement (19).

7. Procédé d'utilisation d'un dispositif conforme à la revendication 1, pour déterminer la résistance au cisaillement inter-laminaire et le module de cisaillement de stratifiés en matériau synthétique renforcé par des fibres comprenant les étapes suivantes consistant à :
a) positionner un échantillon (9) entre un premier corps de base supérieur (1) mobile ayant une plaque de compression (8) associée et un second corps de base inférieur (2) fixe ayant une plaque de compression (7) associée,
b) régler le plan de cisaillement (26) par rotation dans un sens ou dans l'autre des tiges filetées (14, 15), les rails de guidage latéraux (12, 13) se déplaçant perpendiculairement au plan de cisaillement (26) avec les plaques d'appui (10, 11),
c) appliquer la force de contrôle (F) et mesurer simultanément la course de déplacement (s) et la force de contrôle (F),
d) déterminer la résistance au cisaillement inter-laminaire à partir de la force de contrôle (F) maximum en fonction de la surface de l'échantillon cisaillé,
e) déterminer le module de cisaillement à partir de la pente de la courbe contrainte de cisaillement-cisaillement qui est déduite de manière continue de la courbe force de contrôle-course de déplacement.
